# EUROPEAN PATENT APPLICATION

(11) **EP 4 512 485 A1**
(43) Date of publication of application: **26.02.2025**
(21) Application number: 23193381.3
(22) Date of filing: 25.08.2023
(51) Int. Cl.: A61Q 5/02, A61Q 5/12, A61Q 11/00, A61Q 15/00, A61Q 19/00, A61Q 19/10, A61K 8/34, A61K 8/63, A61K 8/68, A61K 8/365, A61K 8/37

(54) **COMPOSITION COMPRISING CERAMIDE, GLYCEROL AND ORGANIC ACID**

(71) Applicant: Evonik Operations GmbH, 45128 Essen (DE); Evonik Dr. Straetmans GmbH, DE-22143 Hamburg (DE)
(72) Inventor: MACZKIEWITZ, Ursula, 45219 Essen (DE); BLASKO_BEGOHIN, heike, 45359 Essen (DE); BRANDT, Kathrin Daniela, 40476 Düsseldorf (DE); MAUS, Lisa, 47228 Duisburg (DE); SCHIEWE, Annemarie, 22149 Hamburg (DE)
(74) Representative: Evonik Patent Association

(57) **Abstract**

The invention relates to a composition comprising at least one ceramide, glycerol and organic acid, a process for producing a formulation comprising at least one ceramide and the use of glycerol at high concentrations for stabilization of a ceramide-containing composition.

## Description

### Field of the invention

The invention relates to a composition comprising at least one ceramide, glycerol and organic acid, a process for producing a formulation comprising at least one ceramide and the use of glycerol at high concentrations for stabilization of a ceramide-containing composition.

### Prior art

Ceramides are used in many cosmetic formulations. However, their low solubility and strong tendency to recrystallization complicate the stable incorporation into cosmetic formulations. Especially at temperatures just above freezing, formulations often suffer from inhomogeneities.

In addition, microbial stabilization of ceramide-containing formulations is challenging since preservatives regularly adversely affect the physical stability of formulations.

The prior art addresses this problem by various delivery forms.

For instance, WO1998053797 describes encapsulated water-insoluble active ingredients having amphiphilic character, with a content of water and at least one surfactant from the group of esters of long-chain carboxylic acids with carboxylic acids comprising hydroxyl groups or salts thereof and esters of long-chain carboxylic acids with polyalcohols, in which ceramides may be the water-insoluble active ingredients.

WO1999029293 describes a composition for topical application, comprising a combination of a free sphingoid base and a ceramide.

JP2008297301 describes ceramides and phytosterol derivatives in triglyceride-containing oil solutions in liquid form for incorporation into dermatological preparations.

The object of the invention was to provide compositions comprising ceramides which have excellent microbial and physical stability.

### Description of the invention

It has been found that, surprisingly, the compositions described below are able to solve the problem addressed by the invention.

The present invention therefore relates to a composition comprising at least one ceramide, high amounts of glycerol and organic acid.

The invention further relates to a process for producing a cosmetic or pharmaceutical formulation and to the use of glycerol for stabilization of a ceramide-containing composition.

An advantage of the present invention is that the compositions according to the invention have increased storage stability and are thus slower to change in their nature overtime, particularly with regard to their viscosity, compared to ceramide-containing compositions according to the prior art. Another advantage of the present invention is that the compositions according to the invention tolerate a higher number of freeze-thaw steps without significant loss of viscosity, compared to ceramide-containing compositions according to the prior art.

One advantage of the composition according to the invention is that the composition in formulations has superior sensory properties which lead to an improved skin feel and/or hair feel.

It is a further advantage of the composition according to the invention that the composition in formulations has improved distributability compared to the individual components.

It is a further advantage of the composition according to the invention that the composition in formulations has improved absorption compared to the individual components.

It is a further advantage of the composition according to the invention that the composition in formulations has reduced oiliness compared to the individual components.

It is a further advantage of the composition according to the invention that the composition in formulations has reduced waxiness compared to the individual components.

It is a further advantage of the composition according to the invention that the composition in formulations has improved glidability compared to the individual components.

It is a further advantage of the composition according to the invention that the composition in formulations has reduced tackiness compared to the individual components.

It is a further advantage of the composition according to the invention that the composition in formulations has improved silkiness/velvetiness compared to the individual components.

A further advantage of the composition according to the invention is that the composition improves skin roughness and skin smoothness more potently than ceramide-containing compositions according to the prior art.

In the context of the present invention, the term "ceramide" is understood to mean acylated sphingoid bases, where the sphingoid bases are preferably selected from sphingosine, sphinganine, 6-hydroxysphingosine and phytosphingosine.

The "pH" in connection with the present invention is defined as the value which is measured for the relevant composition at 22°C after stirring for five minutes using a pH electrode calibrated in accordance with ISO 4319 (1977).

Unless stated otherwise, all percentages (%) given are percentages by mass.

The present invention therefore relates to a composition comprising
A) at least one ceramide,
B) glycerol and
C) at least one organic acid comprising 2 to 12 carbon atoms or a salt thereof, characterized in that
the glycerol is comprised in an amount of 15 wt.-% to 75 wt.-%, preferably of 20 wt.-% to 60 wt.-%, more preferably of 25 wt.-% to 50 wt.-%, most preferably of 27 wt.-% to 40 wt.-%, wherein the percentages refer to the total composition.

It is preferred in accordance with the invention that the composition according to the invention is characterized in that the organic acid is selected from the group of citric acid, formic acid, tartaric acid, oxalic acid, malic acid, maleic acid, succinic acid, mandelic acid, glycolic acid, isobutyric acid, butyric acid, gallic acid, acetic acid, anisic acid, lactic acid and levulinic acid, preferably citric acid, glycolic acid, tartaric acid, malic acid and lactic acid, most preferably lactic acid.

Preferred compositions according to the invention are characterized in that the organic acid is comprised in an amount of 0.01 wt.-% to 3.0 wt.-%, preferably of 0.1 wt.-% to 2.5 wt.-%, more preferably of 0.15 wt.-% to 2.0 wt.-%, most preferably of 0.2 wt.-% to 1.5 wt.-%, wherein the percentages refer to the total composition.

When determining the mass of component C) only the mass of the acid form is regarded; if salts of the organic acid are comprised the counter ions are disregarded.

It is preferred in accordance with the invention that the composition according to the invention is characterized in that said composition has a pH in the range of 1.5 to 6.5, preferably 2.0 to 5.4, particularly preferably 2.5 to 4.5, most preferably 3.1 to 3.9.

A preferred composition according to the invention is characterized in that said composition comprises at least two ceramides, preferably at least three ceramides, particularly preferably precisely three ceramides.

It is preferred in accordance with the invention that the composition according to the invention is characterized in that said at least one ceramide is selected from the group comprising ceramide NP, ceramide AP, ceramide EOP, ceramide NDS (also known as Ceramide NG), ceramide ADS, ceramide EODS, ceramide NS, ceramide AS, ceramide EOS, ceramide NH, ceramide AH and ceramide EOH, preferably selected from the group comprising ceramide NP, ceramide AP, ceramide NS, ceramide EOP and ceramide EOS.

A preferred composition according to the invention is characterized in that it additionally comprises D) cholesterol and/or at least one cholesterol derivative selected from the group comprising cholesterol sulphate, cholesterol hydrogen succinate and 7-dehydrocholesterol.
Component D) preferably is comprised in a total amount of 0.1% by weight to 3.0% by weight, preferably 0.1% by weight to 2.0% by weight, particularly preferably 0.2% by weight to 1.0% by weight, where the percentages by weight refer to the total composition,

It is preferred in accordance with the invention that the composition according to the invention is characterized in that it comprises
E) at least one sphingoid base, preferably selected from the group comprising sphingosine, sphinganine, 6-hydroxysphingosine, N-acetylphytosphingosine and phytosphingosine, especially phytosphingosine.

Component E) is preferably present according to the invention in an amount of 0.01% by weight to 2.0% by weight, preferably 0.02% by weight to 1% by weight, particularly preferably 0.02% by weight to 0.8% by weight, based on the total composition according to the invention.

According to the invention, the ratio by weight of component A) to component D) in the composition according to the invention is preferably from 1: 1 to 1: 0.01, preferably from 1: 0.8 to 1: 0.02, particularly preferably from 1: 0.7 to 1: 0.05.

A preferred composition according to the invention is characterized in that said composition comprises water, preferably in the range of 20% by weight to 65 % by weight, based on the total composition.

The present invention further relates to a process for producing a cosmetic or pharmaceutical formulation comprising A) at least one ceramide, comprising the process steps of:
a) providing a composition according to the instant invention;
b) mixing the composition with water and at least one selected from cosmetical and pharmaceutical active components.

The cosmetical and pharmaceutical active components of step b) are, of course different from any component A) to E) comprised in the composition of the instant invention.

Preferred compositions according to the instant invention are preferably used in the context of the process according to the instant invention.

A preferred process according to the instant invention is characterized in that said process comprises
c) emulsification of the formulation.

In consequence, cosmetic or pharmaceutical formulation produced by the process according to the instant invention preferably is an emulsion, in particular an oil-in-water emulsion.

To facilitate the emulsification step c) of the process according to the instant invention it is preferred in accordance with the invention that the at least one emulsifier is present in process step c).

The term "emulsifier" in the context of the present invention is understood to mean organic substances having surface-active properties which may have the ability to lower the surface tension of water at 20°C and at a concentration of 0.5% by weight, based on the total composition, to less than 45 mN/m, and for which there is no pH between 2 and 12, in which at 20°C, at least 50 mol% of the molecules are ionically charged. Thus, these substances are overall mostly uncharged from the outside at all pH values between 2 and 12.

The surface tension is determined here by the ring method in accordance with du Noüy at 20°C. Emulsifiers preferably present are selected from the group of acyl lactylates (especially sodium lauroyl lactylate), ethoxylated fatty alcohols (especially ceteareth-25), ethoxylated fatty acids (especially PEG_1 00 stearates), polyglycerol esters (especially polyglyceryl-3 methylglucose distearate, polyglyceryl-6 stearate, polyglyceryl-6 behenate, polyglyceryl-10 stearate), glycerol esters, (especially glyceryl stearate citrate), ethoxylated sorbitan esters, lecithin, hydrogenated lecithin, phosphatidylcholin and hydrogenated phosphatidylcholin.

The term "acyl lactylate" in the context of the present invention is understood to mean salt-form reaction products of fatty acid with lactic acid and/or polylactic acid.

A preferred process according to the instant invention is characterized in that said process comprises
d) adjusting the pH of the formulation to a range of 4.0 to 8.0, preferably 4.5 to 7.4 particularly preferably 5.0 to 7.2.

The formulations prepared by the process according to the invention are in particular physically stable formulations. In the context of the present invention, the term "physically stable formulations containing at least one ceramide" is understood in particular to mean formulations which in particular do not exhibit any crystallization of the ceramides and the sphingoid base and no separation or inhomogeneity after six months of storage at 25°C.

The present invention further relates to the use of glycerol, preferably in an amount of 15 wt.-% to 75 wt.-%, preferably of 20 wt.-% to 60 wt.-%, more preferably of 25 wt.-% to 50 wt.-%, most preferably of 27 wt.-% to 40 wt.-%, wherein the percentages refer to the total composition, for physical stabilization of a ceramide-containing composition, in particular with regard to homogeneity, and/or for preventing crystallization of at least one ceramide in a composition and/or for microbial stabilization of a ceramide-containing composition, in particular with respect to at least one selected from *S*. *aureus, P. aeruginosa, E. coli, C. albicans* and A. *brasiliensis.*

Preferably the ceramide-containing composition in the use of the instant invention comprises at least one organic acid comprising 2 to 12 carbon atoms or a salt thereof.

In the context of the use according to the instant invent the ceramide-containing composition are preferably equal to the preferred compositions according to the instant invention, especially regarding components A) and C).

The examples adduced hereinafter describe the present invention by way of example, without any intention that the invention, the scope of application of which is apparent from the entirety of the description and the claims, be restricted to the embodiments specified in the examples.

### Examples:

### Example 1: Physical stability

**Table 1: Examples of formulations: * according to the invention.**

| Phase | Example | No 1 | No 2* | No 3* | No 4* | No 5* | No 6* | No 7* | No 8* |
|---|---|---|---|---|---|---|---|---|---|
| A | Polyglyceryl-10 Stearate | | | 6.0% | 4.0% | | | | |
| A | Polyglycery-6 Behenate | | | 2.0% | 2.0% | | | | |
| A | Sodium Lauroyl Lactylate | 8.0% | 8.0% | | | | | | |
| A | Hydrogenated Lecithin | | | | | 5.0% | | | |
| A | Lecithin | | | | | | 5.0% | | |
| A | Phosphatidylcholin | | | | | | | 4.0% | |
| A | Hydrogenated Phosphatidylcholin | | | | | | | | 3.5% |
| A | Cetearyl Alcohol | 1.0 | 1.0 | 0.4% | 0.5% | 1.0% | 0.5% | 1.0% | 1.3% |
| A | Glyceryl Stearate | | | 0.4% | 0.5% | | 1.5% | 1.0% | 0.7% |
| A | Ceramide NP | | | 2.0% | 0.5% | 0.3% | 1.5% | 1.0% | 1.0% |
| A | Ceramide AP | 0.5% | 0.5% | | 0.5% | 0.3% | | 0.5% | |
| A | Ceramide NS | 0.5% | 0.5% | | 0.5% | | 0.5% | | |
| A | Ceramide NDS | | | | | 0.3% | | | 1.0% |
| A | Ceramide EOP | | | | 0.1% | 0.05% | | 0.1% | |
| A | Ceramide EOS | 0.1% | 0.1% | | | 0.05% | | | |
| A | Phytosphingosine | | | 0.5% | | 0.3% | | 0.7% | |
| A | Sphinganine | | | | | 0.3% | | | |
| A | Hydroxybutyroyl Phytosphingosine | | | | | | 0.5% | | |
| A | Salicyloyl Phytosphingosine | 0.5% | 0.5% | | | | | | |
| A | Cholesterol | 0.4% | 0.4% | 0.5% | 0.7% | 0.4% | 0.3% | 0.5% | 0.3% |
| A | Behenic Acid | | | 0.5% | | | 0.2% | | 0.7% |
| A | Carnauba Acid Wax | 0.5% | 0.5% | | 0.5% | 0.5% | 0.4% | 0.5% | |
| A | Triethyl Citrate | | | 2.5% | 1.8% | | | | |
| B | Glycerin | 3.0% | 35.0% | 30.0% | 33.0% | 35.0% | 30.0% | 45.0% | 40.0% |
| B | Water | 84.2% | 52.8% | 54.6% | 54.8% | 55.8% | 59.0% | 44.9% | 50.8% |
| C | Lactic Acid | 0.1% | 0.7% | 0.6% | 0.6% | 0.7% | 0.6% | 0.8%% | 0.7% |
| D | Ethylhexylglycerin | 1.2% | | | | | | | |

Preparation of the example formulations: phase A and B are heated separately to 85-90 °C. Then phase A is added to phase B under stirring. After the complete addition of phase A the formulation is homogenized for 1-5 minutes using an Ultra Turrax or another homogenizer based on a similar homogenization technique. Then the formulation is cooled to 30-35 °C under stirring and phase C (Lactic Acid) is added. If existing add phase D and stirr until it is homogenously dispersed.

The formulations were stored for 6 months at room temperature and controlled regarding homogeneity, phase separation and recrystallization of the ceramides or other sphingolipids. The control of the homogeneity as well as of the phase separation was carried out visually using the following scores:
Homogeneity:
   H0 = completely homogenous, no inhomogeneity
   H1 = slightly inhomogeneous
   H2 = homogeneous
Separation:
   S0 = no separation
   S1 = slight separation
   S2 = significant separation
   S3 = complete separation

The recrystallization of ceramides and other sphingolipids was controlled using a microscope and the following criteria:
K0 = no crystals visible
K1 = a few number of crystals visible
K2 = significant number of crystals visible
K3 = a very high number of crystals visible

**Table 2: Result of stability test for examples no 1-8.**

| Example | No 1 | No 2* | No 3* | No 4* | No 5* | No 6* | No 7* | No 8* |
|---|---|---|---|---|---|---|---|---|
| Homogeneity | H1 | H0 | H0 | H0 | H0 | H0 | H0 | H0 |
| Separation | S2 | S0 | S0 | S0 | S0 | S0 | S0 | S0 |
| Recrystallization | K3 | K0 | K0 | K0 | K0 | K0 | K0 | K0 |

Regarding homogeneity, phase separation and recrystallization the formulations of example 2-8 were completely stable for at least 6 months at room temperature while the formulation of example 1 which is not in accordance with the present invention was slightly inhomogeneous, showed a significant separation and a strong recrystallization after six months storage at room temperature.

### Example 2: Microbial stability

The formulations No 1, 2 and 3 were tested in a preservation challenge test. The test was performed according to the European Pharmacopoeia 2011:5.1.3. A typical preservation challenge test is performed as follows:
The product is inoculated with defined, single microorganisms and stored under defined conditions. At fixed time points the number of microorganisms (given as colony forming units CFU) is detected. If the preservation challenge test should be fulfilled a specific decrease of the different microorganisms is necessary to fulfill criterion A or B regarding preservation efficacy. For raw material it is common sense that criterion B is sufficient.

Table 3 shows bacteria and fungi which are tested and the number of log steps that has to decrease at a specific time point to fulfill criterion A resp. B. A formulation passes the preservation challenge test if for all tested microorganisms at least criterion B is achieved.

**Table 3: Number of log steps for the specific microorganism which has to be reduced at the given time points to pass the preservation challenge test.**

| | Criterion | 2 days | 7 days | 14 days | 28 days |
|---|---|---|---|---|---|
| *Escherichia coli* | A | 2 | 3 | | No increase |
| | B | | | 3 | No increase |
| *Pseudomonas aeruginosa* | A | 2 | 3 | | No increase |
| | B | | | 3 | No increase |
| *Staphylococcus aureus* | A | 2 | 3 | | No increase |
| | B | | | 3 | No increase |
| *Candida albicans* | A | | | 2 | No increase |
| | B | | | 1 | No increase |
| *Aspergillus brasiliensis* | A | | | 2 | No increase |
| | B | | | 1 | No increase |

The following table shows the result of the preservation challenge test of formulation No. 1 - 3

**Table 4: result of preservation challenge test of example no 1**

| No. 1 | Start | 2 days | 7 days | 14 days | 28 days | Assessment |
|---|---|---|---|---|---|---|
| *Escherichia coli* | 450000 | 42000 | 45000 | 30000 | 5000 | failed |
| *Pseudomonas aeruginosa* | 500000 | 20000 | 5000 | 400 | <10 | B |
| *Staphylococcus aureus* | 410000 | 400 | 200 | <10 | <10 | A |
| *Candida albicans* | 390000 | 120000 | 15000 | 4500 | 100 | B |
| *Aspergillus brasiliensis* | 390000 | 40000 | 35000 | 38000 | 40000 | failed |

**Table 5: result of preservation challenge test of example no 2**

| No. 2* | Start | 2 days | 7 days | 14 days | 28 days | Assessment |
|---|---|---|---|---|---|---|
| *Escherichia coli* | 450000 | 1000 | 100 | <10 | <10 | A |
| *Pseudomonas aeruginosa* | 500000 | <10 | <10 | <10 | <10 | A |
| *Staphylococcus aureus* | 410000 | <10 | <10 | <10 | <10 | A |
| *Candida albicans* | 390000 | <10 | <10 | <10 | <10 | A |
| *Aspergillus brasiliensis* | 390000 | 10000 | 1000 | 100 | <10 | A |

**Table 6: result of preservation challenge test of example no 3**

| No. 3* | Start | 2 days | 7 days | 14 days | 28 days | Assessment |
|---|---|---|---|---|---|---|
| *Escherichia coli* | 450000 | <10 | <10 | <10 | <10 | A |
| *Pseudomonas aeruginosa* | 500000 | <10 | <10 | <10 | <10 | A |
| *Staphylococcus aureus* | 410000 | <10 | <10 | <10 | <10 | A |
| *Candida albicans* | 390000 | <10 | <10 | <10 | <10 | A |
| *Aspergillus brasiliensis* | 390000 | 1000 | 50 | <10 | <10 | A |

Example 1 did not pass the preservation challenge test because *Escherichia coli* and *Aspergillus brasiliensis* did not fulfill at least criterion B. The examples 2 and 3 which are according to the present invention passed the preservation challenge test and even fulfilled the stronger criterion A.

### Example 3: Example formulations

Each of the formulations below was produced 7 times while incorporating formulations No. 2 to 8 into them, depicted as "No. X" below.

### Face cream I

| Phase | | | | | | | | | |
|---|---|---|---|---|---|---|---|---|---|
| A | TEGO^{®} Care 450 (Polyglyceryl-3 Methylglucose Distearate) | 3.0 | 3.0 | 3.0 | 3.0 | 3.0 | 3.0 | 3.0 | 3.0 |
| | TEGIN^{®} M Pellets MB (Glyceryl Stearate) | 2.0 | 2.0 | 2.0 | 2.0 | 2.0 | 2.0 | 2.0 | 2.0 |
| | TEGO^{®} Alkanol 1618 MB (Cetearyl Alcohol) | 1.0 | 1.0 | 1.0 | 1.0 | 1.0 | 1.0 | 1.0 | 1.0 |
| | TEGOSOFT^{®} OS (Ethylhexylstearate) | 8.0 | 8.0 | 8.0 | 8.0 | 8.0 | 8.0 | 8.0 | 8.0 |
| | TEGOSOFT^{®} OER MB (Oleyl Erucate) | 5.0 | 5.0 | 5.0 | 5.0 | 5.0 | 5.0 | 5.0 | 5.0 |
| | TEGOSOFT^{®} DC MB (Decyl Cocoate) | 6.0 | 6.0 | 6.0 | 6.0 | 6.0 | 6.0 | 6.0 | 6.0 |
| | SPHINOX^{®} Defenda (Hydroxybutyroyl Phytosphingosine) | | | 0.1 | 0.1 | | | | |
| B | Glycerin | 3.0 | 3.0 | 3.0 | 3.0 | 3.0 | 3.0 | 3.0 | 3.0 |
| | Water | ad 100 | ad 100 | ad 100 | ad 100 | ad 100 | ad 100 | ad 100 | ad 100 |
| | HyaCare^{®} (Sodium Hyaluronate) | | | | | 0.1 | | | |
| | HyaCare^{®} 50 (Hydrolyzed Hyaluronic Acid) | | | | | | 0.1 | | |
| | Dihydroxyacetone | | 1.0 | | 1.0 | | | | |
| C | TEGO^{®} Pep 4-17 (Tetrapeptide-21, Glycerin, Butylene Glycol, Water) | | | | | | | 2.0 | |
| | TEGO^{®} Pep UP (Tetrapeptide-4, Glycerin, Water) | | | | | | | | 2.0 |
| D | No. X | 1.0 | 1.0 | 1.0 | 1.0 | 1.0 | 1.0 | 1.0 | 1.0 |
| E | Verstatil^{®} PC (Phenoxyethanol, Caprylyl Glycol) | 1.0 | 1.0 | 1.0 | 1.0 | 1.0 | 1.0 | 1.0 | 1.0 |

Preparation: Heat phase A and B to 85 °C. Add phase A to B with stirring. Homogenize. Cool down while stirring to 40 °C. Add phase C, D and E. Cool down while stirring to 30 °C.

### Face cream II

| Phase | | | | | | | |
|---|---|---|---|---|---|---|---|
| A | TEGO^{®} Care 450 (Polyglyceryl-3 Methylglucose Distearate) | 3.0 | 3.0 | 3.0 | 3.0 | 3.0 | 3.0 |
| | TEGIN^{®} M Pellets MB (Glyceryl Stearate) | 2.0 | 2.0 | 2.0 | 2.0 | 2.0 | 2.0 |
| | TEGO^{®} Alkanol 1618 MB (Cetearyl Alcohol) | 1.0 | 1.0 | 1.0 | 1.0 | 1.0 | 1.0 |
| | TEGOSOFT^{®} OS (Ethylhexylstearate) | 8.0 | 8.0 | 8.0 | 8.0 | 8.0 | 8.0 |
| | TEGOSOFT^{®} OER MB (Oleyl Erucate) | 5.0 | 5.0 | 5.0 | 5.0 | 5.0 | 5.0 |
| | TEGOSOFT^{®} DC MB (Decyl Cocoate) | 6.0 | 6.0 | 6.0 | 6.0 | 6.0 | 6.0 |
| | SPHINOX^{®} Defenda (Hydroxybutyroyl Phytosphingosine) | | 0.1 | | | | |
| B | Glycerin | 3.0 | 3.0 | 3.0 | 3.0 | 3.0 | 3.0 |
| | Water | ad 100 | ad 100 | ad 100 | ad 100 | ad 100 | ad 100 |
| | HyaCare^{®} (Sodium Hyaluronate) | | | 0.1 | | | |
| | HyaCare^{®} 50 (Hydrolyzed Hyaluronic Acid) | | | | 0.1 | | |
| C | No. X | 2.0 | 2.0 | 2.0 | 2.0 | 2.0 | 2.0 |
| D | RHEASOME^{®} Ceraboost (Water/Aqua; Glycerin; Lecithin; Glycolipids; Ceramide NP; Phytosphingosine; Cholesterol; Benzyl Alcohol; Benzoic Acid; Dehydroacetic Acid; Sodium Benzoate; Tocopherol) | | | | | 4.0 | |
| | InnuMax^{®} Advanced Retinol (Water/Aqua; Caprylic/Capric Triglyceride; Glycerin; Polysorbate 20; Retinol; Inulin Lauryl Carbamate; Behentrimonium Chloride; Sucrose Laurate) | | | | | | 5.0 |
| E | Verstatil^{®} PC (Phenoxyethanol, Caprylyl Glycol) | 1.0 | 1.0 | 1.0 | 1.0 | 1.0 | 1.0 |

Preparation: Heat phase A and B to 85 °C. Add phase A to B with stirring. Homogenize. Cool down while stirring to 40 °C. Add phase C, D and E. Cool down while stirring to 30 °C.

### Face cream III

| Phase | | | | | | | |
|---|---|---|---|---|---|---|---|
| A | TEGO^{®} Care 450 (Polyglyceryl-3 Methylglucose Distearate) | 3.0 | 3.0 | 3.0 | 3.0 | 3.0 | 3.0 |
| | TEGIN^{®} M Pellets MB (Glyceryl Stearate) | 2.0 | 2.0 | 2.0 | 2.0 | 2.0 | 2.0 |
| | TEGO^{®} Alkanol 1618 MB (Cetearyl Alcohol) | 1.0 | 1.0 | 1.0 | 1.0 | 1.0 | 1.0 |
| | TEGOSOFT^{®} OS (Ethylhexylstearate) | 8.0 | 8.0 | 8.0 | 8.0 | 8.0 | 8.0 |
| | TEGOSOFT^{®} OER MB (Oleyl Erucate) | 5.0 | 5.0 | 5.0 | 5.0 | 5.0 | 5.0 |
| | TEGOSOFT^{®} DC MB (Decyl Cocoate) | 6.0 | 6.0 | 6.0 | 6.0 | 6.0 | 6.0 |
| | SPHINOX^{®} Defenda (Hydroxybutyroyl Phytosphingosine) | | 0.1 | | | | |
| B | Glycerin | 3.0 | 3.0 | 3.0 | 3.0 | 3.0 | 3.0 |
| | Water | ad 100 | ad 100 | ad 100 | ad 100 | ad 100 | ad 100 |
| | HyaCare^{®} (Sodium Hyaluronate) | | | 0.1 | | | |
| | HyaCare^{®} 50 (Hydrolyzed Hyaluronic Acid) | | | | 0.1 | | |
| C | No. X | 2.5 | 2.5 | 2.5 | 2.5 | 2.5 | 2.5 |
| D | Verstatil^{®} PC (Phenoxyethanol, Caprylyl Glycol) | 1.0 | 1.0 | 1.0 | 1.0 | 1.0 | 1.0 |

Preparation: Heat phase A and B to 85 °C. Add phase A to B with stirring. Homogenise. Cool down while stirring to 40 °C. Add phase C and D. Cool down while stirring to 30 °C.

### O/W lotion, skin fluid

| Phase | Ingredients (INCI) | | | |
|---|---|---|---|---|
| A | TEGO^{®} Care PBS 6 MB (Polyglyceryl-6 Stearate (and) Polyglyceryl-6 Behenate) | 3.0 | 3.0 | 3.0 |
| | TEGO^{®} Alkanol 1618 MB (Cetearyl Alcohol) | 1.0 | 1.0 | 1.0 |
| | TEGIN^{®} M Pellets MB (Glyceryl Stearate) | 1.0 | 1.0 | 1.0 |
| | TEGOSOFT^{®} MM (Myristyl Myristate) | 1.0 | 1.0 | 1.0 |
| | TEGOSOFT^{®} OS (Ethylhexyl Stearate) | 5.0 | 5.0 | 5.0 |
| | Isopropyl Myristate | 3.5 | 3.5 | 3.5 |
| | ABIL^{®} 350 (Dimethicone) | 2.0 | 2.0 | 2.0 |
| | Mineral Oil (Paraffinum Liquidum) | 5.0 | 5.0 | 5.0 |
| B | Water | ad 100 | ad 100 | ad 100 |
| C | Glycerin | 3.0 | 3.0 | 3.0 |
| | Keltrol CG SFT (Xanthan Gum) | 0.5 | 0.5 | 0.5 |
| | Water | 10.0 | 10.0 | 10.0 |
| D | No. X | 1.5 | 1.5 | 1.5 |
| E | TEGO^{®} Natural Betaine (Betaine) | | 2.0 | |
| | Water | | 2.0 | |
| | LACTIL^{®} (Sodium Lactate; Sodium PCA; Glycine; Fructose; Urea; Niacinamide; Inositol; Sodium Benzoate; Lactic Acid) | | | 2.0 |
| F | Verstatil^{®} TBO (Triethyl Citrate; Caprylyl Glycol; Benzoic Acid) | 1.0 | 1.0 | 1.0 |

Preparation:
Heat phase A and B to 85 °C. Add phase A to B with stirring. Homogenise. Cool with gentle stirring to approx. 40 °C and add phase C. Homogenise for a short time. Add phase D, E and F step by step.

### Enjoy The Sun Lotion, SPF 50

| Phase | Ingredients (INCI) | |
|---|---|---|
| A | TEGO^{®} Care PBS 6 (Polyglyceryl-6 Stearate (and) Polyglyceryl-6 Behenate) | 4.00 |
| | dermofeel sensolv (Isoamyl Laurate) | 8.00 |
| | TEGIN^{®} M Pellets MB (Glyceryl Stearate | 0.25 |
| | TEGO^{®} Alkanol 1618 MB (Cetearyl Alcohol) | 0.25 |
| | dermofeel^{®} Toco 70 non GMO (Tocopherol; Helianths Anuus (Sunflower) Seed Oil) | 0.20 |
| | Bis-Ethyhexyloxyphenol Methoxyphenyl Triazine | 6.00 |
| | Diethylamino Hydroxybenzoyl Hexyl Benzoate | 6.00 |
| | Ethylhexyl Salicylate | 5.00 |
| | Ethylhexyl Triazone | 2.00 |
| B | Water | ad 100 |
| | Glycerin | 3.00 |
| | Disodium EDTA | 0.05 |
| C | Keltrol CG SFT (Xanthan Gum) | 0.50 |
| D | Water | 10.67 |
| | Euxolex 232 (Phnylbenzimidazole Sulfonic Acid) | 3.00 |
| | Tromethamine | 1.33 |
| E | No. X | 3.0 |
| F | Perfume | q.s |
| | Tromethamine, 30% in water | q.s. |
| G | Verstatil^{®} BOB (Benzyl Alcohol; Caprylyl Glycol; Benzoic Acid) | 1.20 |

Preparation:
Heat phase A and B separately to approx. 85 °C. Add phase A to B. Homogenize. Cool with gentle stirring to approx. 50 °C, then add phase C. Homogenise. Cool with gentle stirring to approx. 35 °C, then add phase D, E, F and G.

### Microemulsion

| Phase | Ingredients (INCI) | |
|---|---|---|
| A | Water | ad 1009 |
| B | VARISOFT^{®} PATC (Palmitamidopropyltrimonium Chloride) | 2.5 |
| | Glycerin | 3.0 |
| C | LACTIL^{®} (Sodium Lactate; Sodium PCE; Fructose; Urea; Niacinamide; Inositol; Sodium Benzoate; Lactic Acid) | 2.0 |
| D | TEGO^{®} Cosmo C 100 (Creatine) | 0.5 |
| E | No. X | 0.5 |
| F | Verstatil^{®} SL (Aqua; Sodium Levulinate; Potassium Sorbate) | 2.0 |
| G | Lactic Acid (10% in water) | q.s |

Preparation:
Add phase B to phase A slowly while stirring. Add phase C, D, E and F step by step. Adjust pH with phase G to pH 5-5.5.

### Facial Cleanser

| Phase | Ingredients (INCI) | |
|---|---|---|
| A | TEGOSOFT^{®} PC 31 (Polyglyceryl-3 Caprate) | 2.50 |
| | TEGO^{®} Solve 61 MB (Polyglyceryl-6 Caprate; Polyglyceryl-3 Cocoate; Polyglyceryl-4 Caprate; Polyglyceryl-6 Ricinoleate) | 2.50 |
| | SPHINGONY^{®} (Sphinganine) | 0.10 |
| | dermosoft^{®} Pentiol eco (Pentylene Glycol) | 5.50 |
| | TAGAT CH 40 (PEG-40 Hydrogenated Castor Oil) | 6.00 |
| B | Water | ad 100 |
| | Keltrol CG-SFT (Xanthan Gum) | 0.20 |
| C | No. X | 0.8 |
| D | Lactic Acid (10% in water) | q.s. |

### Preparation

Heat phase A up to 85 °and stirr until it is clear. Add phase B to A slowly with gentle stirring. Cool down to 30 °C. Add phase C. Adjust pH with phase D between 5.0-5.5.

### Shampoo

| Phase | Ingredients (INCI) | |
|---|---|---|
| A | Sodium Laureth Sulfate, 28% | 32.00 |
| | dermofeel^{®} sensolv MB (Isoamyl Laurate) | 1.00 |
| | dermofeel^{®} G10 LW 70 MB (Polyglyceryl-10 Laurate; Aqua; Citric Acid) | 4.00 |
| | Water | ad 100 |
| | TEGO^{®} Betain P 50 SG (Cocoamidopropyl Betaine) | 10.00 |
| | dermofeel^{®} quadegra (Aqua; Starch Hydroxypropyl trimonium Chloride; Urea; Sodium Lactate; Lactic Acid; Sodium Chloride; Glycerin; Levulinic Acid; p-Anisic Acid) | 1.50 |
| | No. X | 0.3 |
| | Verstatil^{®} BP (Phenoxyethanol; Benzoic Acid) | 0.80 |
| | ANTIL^{®} 200 MB (PEG-200 Hydrogenated Glyceryl Palmate; PEG-7 Glyceryl Cocoate) | 4.00 |

Preparation:
Mix Sodium Laureth Sulfate, dermofeel sensolv MB, dermofeel G10 LW70 MB until the solution is clear, heat if necessary. Add the other ingredients step by step in the given order. Cool down while stirring to 30 °C.

### Oral Care (Mouth Wash)

| Phase | Ingredients (INCI) | |
|---|---|---|
| A | RHEANCE^{®} One (Glycolipids) | 0.20 |
| | Flavor Mintfresh | 0.20 |
| | Water | ad 100 |
| | Sodium Saccharine 450 | 0.07 |
| | Sorbitol, 70% | 4.00 |
| | No. X | 0.20 |
| | Verstatil^{®} SL non GMO (Aqua; Sodium Levulinate; Potassium Sorbate) | 2.00 |

### Preparation

Dissolve the flavour in RHEANCE One. Add the water slowly while stirring, then add the remaining ingredients in the given order.

### Deo Formulation (Natural Milky Deo Roll-On

| Phase | Ingredients (INCI) | |
|---|---|---|
| A | Water | ad 100 |
| | Glycerin | 5.00 |
| | Trisodium Citrate Dihydrate (Sodium Citrate) | 0.30 |
| | Xanthan Gum FNCSP-PC (Xanthan Gum) | 0.90 |
| | Genuvisco Carrageenan CG131 (Carrageenan) | 0.10 |
| B | dermosoft^{®} decalact delo MB (Sodium Caproyl/Lauroyl Lactylate; Triethyl Citrate; Salvia Officinalis (Sage) Oil) | 0.30 |
| C | dermofeel^{®} GSC SG (Glyceryl Stearate Citrate) | 0.40 |
| | Apricot Kernel Oil, refined (Prunus Armeniaca Kernel Oil) | 0.50 |
| | Sunflower Oil, pressed, organic (Helianthus annuus seed oil) | 0.50 |
| | TEGODEO^{®} PY 88 G (Zinc Ricinoleate) | 1.00 |
| | dermofeel^{®} TEC eco (Triethyl Citrate) | 5.00 |
| | dermosoft^{®} G 3 CY MB (Polyglyceryl-3 Caprylate) | 1.00 |
| D | No. X | 1.00 |
| | Perfume | 0.30 |
| | dermosoft^{®} 7GA MB (Aqua; Glycerin; Heptylglycerin; Sodium Anisate) | 2.60 |
| E | Citric Acid (20% in water) | 1.47 |

### Preparation

Heat phase A up to 80 °C and disperse phase B in A. Heat phase C up to 85 °C separately. Add phase C into phae A/B. Cool down to 35 °C and add phase D in the given order under slowly stirring. Add phase E to adjust the pH value.

### Shower Gel

| Phase | Ingredients (INCI) | |
|---|---|---|
| A | Sodium Laureth Sulfate, 28% | 37.00 |
| | Verstatil^{®} PC (Phenoxyethanol; Caprylyl Glycol) | 1.00 |
| | Water | ad 100 |
| | ANTIL^{®} Soft SC MB (Sorbitan Sesquicaprylate) | 1.00 |
| | REWOTERIC^{®} AM C MB (Sodium Cocoamphoacetate) | 9.00 |
| | TEGO^{®} Betain 810 MB (Capryl/Capramidopropyl Betaine) | 7.60 |
| | Citric Acid (30% in water) | 1.30 |
| | REWODERM^{®} LI S 80 MB (PEG-200 Hydrogenated Glyceryl Palmate; PEG-7 Glyceryl Cocoate) | 0.50 |
| | No. X | 0.50 |
| | Sodium Chloride | 0.20 |

### Preparation

Mix the ingredients slowly step by step in the given order while stirring.

### Facial Masque

| Phase | Ingredients (INCI) | | | |
|---|---|---|---|---|
| A | TEGO^{®} Care PBS 6 (Polyglyceryl-6 Stearate (and) Polyglyceryl-6 Behenate) | 4.00 | 4.00 | 4.00 |
| | Stearyl Alcohol | 2.00 | 2.00 | 2.00 |
| | TEGIN^{®} M Pellets MB (Glyceryl Stearate) | 1.00 | 1.00 | 1.00 |
| | TEGOSOFT CR MB (Cetyl Ricinoleate) | 2.00 | 2.00 | 2.00 |
| | TEGOSOFT^{®®} APM (PPG-3 Myristyl Ether) | 12.0 | 12.0 | 12.0 |
| | TEGOSOFT^{®} OP (Ethylhexyl Palmiate) | 10.00 | 10.00 | 10.00 |
| | dermosoft^{®} OMP (Methylpropanediol; Caprylyl Glycol; Phenylpropanol) | 3.50 | 3.50 | 3.50 |
| B | Water | ad 100 | ad 100 | ad 100 |
| | Dihydroxyacetone | | 1.00 | |
| | Erythrulose | | | 0.50 |
| C | Sclerotium Gum | 0.53 | 0.53 | 0.53 |
| | Glycerin | 5.00 | 5.00 | 5.00 |
| | Keltrol CG-SFT (Xanthan Gum) | 0.27 | 0.27 | 0.27 |
| D | TEGO^{®} Renew HA Malto (Maltobionic Acid) | 1.00 | 1.00 | 1.00 |
| | TEGO^{®} Renew HA Lacto (Lactobionic Acid) | 1.00 | 1.00 | 1.00 |
| | Water | 20.00 | 20.00 | 20.00 |
| E | No. X | 1.50 | 1.50 | 1.50 |
| F | Citric Acid (10% in water) | q.s. | q.s. | q.s. |

### Preparation

Heat phase A and B to 85 °C. Add phase A to B with stirring. Homogenize. Cool with gentle stirring to 50 °C. Add phase C at about 50 °C. Homogenize again for a short time. Cool with gentle stirring to appr. 35 °C. Prepare phase D. Add phase D with stirring. Add phase E. Adjust pH with phase F to 4.0-5.0.

### Conditioner for Hair

| Phase | Ingredients (INCI) | |
|---|---|---|
| A | TEGO^{®} Alkanol 1618 MB (Cetearyl Alcohol) | 6.00 |
| | VARISOFT^{®} BT 85 Pellets (Behentrimonium Chloride) | 2.50 |
| | VARISOFT^{®} EQ 100 (Bis-(Isosearoyl/Oleoyl Isopropyl) Dimonium Methosulfate) | 1.00 |
| | Hairflux^{®} (Olea Europaea (Olive) Fruit Oil; Ricinus Communis (Castor) Seed Oil; Ceramide NG | 1.00 |
| B | ABIL^{®} Quat 3272 (Quaternium-80) | 2.00 |
| | Glycerin | 2.00 |
| | Water | ad 100 |
| C | No. X | 1.00 |
| D | Verstatil^{®} PC (Phenoxyethanol; Caprylyl Glycol) | 1.00 |

### Preparation

Heat phase A and B to 85 °C. Add phase B to phase A without stirring. Homogenize. Cool down to 30 °C while stirring. Add phase C and D.

### W/O Emulsion

| Phase | Ingredients (INCI) | |
|---|---|---|
| A | ABIL^{®} EM 90 (Cetyl PEG/PPG-10/1 Dimethicone) | 2.00 |
| | Paracera W 80 (Microcristalline Wax) | 0.50 |
| | Hydrogenated Castor Oil | 0.50 |
| | Octyldodecanol | 7.00 |
| | TEGOSOFT^{®} CT (Caprylic/Capric Triglyceride) | 7.00 |
| | TEGOSOFT^{®} DC MB (Decyl Cocoate) | 7.00 |
| B | Glycerin | 3.00 |
| | Sodium Chloride | 0.50 |
| | Water | ad 100 |
| C | No. X | 1.00 |
| D | Euxyl PE 9010 (Phenoxyethanol; Ethylhexylglycerin) | 1.00 |

### Preparation

Heat phase A to approx. 85 °C. Add phase B (80 °C or room temperature) slowly while stirring. Homogenise for a short time. Cool with gentle stirring below 30 °C and add phase C. homogenise again for a short time.

## Claims

1. Composition comprising
A) at least one ceramide,
B) glycerol and
C) at least one organic acid comprising 2 to 12 carbon atoms or a salt thereof, **characterized in that**
the glycerol is comprised in an amount of 15 wt.-% to 75 wt.-%, preferably of 20 wt.-% to 60 wt.-%, more preferably of 25 wt.-% to 50 wt.-%, most preferably of 27 wt.-% to 40 wt.-%, wherein the percentages refer to the total composition.

2. Composition according to claim 1, **characterized in that** the organic acid is selected from the group of citric acid, formic acid, tartaric acid, oxalic acid, malic acid, maleic acid, succinic acid, mandelic acid, glycolic acid, isobutyric acid, butyric acid, gallic acid, acetic acid, anisic acid, lactic acid and levulinic acid, preferably citric acid, glycolic acid, tartaric acid, malic acid and lactic acid, most preferably lactic acid.

3. Composition according to claim 1 or 2, **characterized in that** the organic acid is comprised in an amount of 0.01 wt.-% to 3.0 wt.-%, preferably of 0.1 wt.-% to 2.5 wt.-%, more preferably of 0.15 wt.-% to 2.0 wt.-%, most preferably of 0.2 wt.-% to 1.5 wt.-%, wherein the percentages refer to the total composition.

4. Composition according to at least one of the preceding claims, **characterized in that** said composition has a pH in the range of 1.5 to 6.5, preferably 2.0 to 5.4, particularly preferably 2.5 to 4.5, most preferably 3.1 to 3.9.

5. Composition according to at least one of the preceding claims, **characterized in that** said composition comprises at least two ceramides, preferably at least three ceramides, particularly preferably precisely three ceramides.

6. Composition according to at least one of the preceding claims, **characterized in that** the at least one ceramide is selected from the group comprising ceramide NP, ceramide AP, ceramide EOP, ceramide NDS, ceramide ADS, ceramide EODS, ceramide NS, ceramide AS, ceramide EOS, ceramide NH, ceramide AH and ceramide EOH, preferably selected from the group comprising ceramide NP, ceramide AP, ceramide NS, ceramide EOP and ceramide EOS.

7. Composition according to at least one of the preceding claims, **characterized in that** it additionally comprises
D) cholesterol and/or at least one cholesterol derivative selected from the group comprising cholesterol sulfate, cholesterol hydrogen succinate and 7-dehydrocholesterol.

8. Composition according to claim 7, **characterized in that** the cholesterol and/or at least one cholesterol derivative is present in a total amount of from 0.1% by weight to 3.0% by weight, preferably from 0.1% by weight to 2.0% by weight, particularly preferably from 0.2% by weight to 1.0% by weight, where the percentages by weight refer to the total composition.

9. Composition according to at least one of the preceding claims, **characterized in that** said composition comprises water, preferably in the range of 20% by weight to 65 % by weight, based on the total composition.

10. Process for producing a cosmetic or pharmaceutical formulation comprising A) at least one ceramide, comprising the process steps of:
a) providing a composition according to at least one of the claims 1 to 9;
b) mixing the composition with water and at least one selected from cosmetical and pharmaceutical active components.

11. Process according to claim 10, **characterized in that** process comprises
c) emulsification of the formulation.

12. Process according to claim 10 or 11, **characterized in that** process comprises
d) adjusting the pH of the formulation to a range of
4.0 to 8.0, preferably 4.5 to 7.4 particularly preferably 5.0 to 7.2.

13. Use of glycerol, preferably in an amount of 15 wt.-% to 75 wt.-%, preferably of 20 wt.-% to 60 wt.-%, more preferably of 25 wt.-% to 50 wt.-%, most preferably of 27 wt.-% to 40 wt.-%, wherein the percentages refer to the total composition, for physical stabilization of a ceramide-containing composition, in particular with regard to homogeneity, and/or for preventing crystallization of at least one ceramide in a composition and/or for microbial stabilization of a ceramide-containing composition, in particular with respect to at least one selected from S. *aureus, P. aeruginosa, E. coli, C. albicans* and A. *brasiliensis.*
